# EUROPÄISCHE PATENTSCHRIFT

(11) **EP 3 496 672 B1**
(45) Veröffentlichungstag und Bekanntmachung des Hinweises auf die Patenterteilung: **03.09.2025**
(21) Anmeldenummer: 17735531.0
(22) Anmeldetag: 05.07.2017
(51) Int. Cl.: A61F 5/01

(54) **GELENK FÜR EINE ORTHOPÄDISCHE EINRICHTUNG**
JOINT FOR AN ORTHOPAEDIC DEVICE
ARTICULATION POUR UN DISPOSITIF ORTHOPÉDIQUE

(30) Priorität: 10.08.2016 DE 102016114834
(43) Veröffentlichungstag der Anmeldung: 19.06.2019
(73) Patentinhaber: Ottobock SE & Co. KGaA, 37115 Duderstadt (DE)
(72) Erfinder: VOGEL, Carsten, 37115 Duderstadt (DE); SCHILLING, Matthias, 37345 Weißenborn-Lüderode (DE)
(74) Vertreter: Gramm, Lins & Partner Patent- und Rechtsanwälte PartGmbB
(86) Internationale Anmeldenummer: PCT/EP2017/066824
(87) Internationale Veröffentlichungsnummer: WO 2018/028891

(56) Entgegenhaltungen:
- US-A1- 2002 169 402
- US-A1- 2006 211 966
- US-A1- 2007 270 976
- US-A1- 2010 022 929

## Beschreibung

Die Erfindung betrifft ein Gelenk für eine orthopädietechnische Einrichtung, das einen ersten Gelenkarm und einen zweiten Gelenkarm, die um eine Schwenkachse schwenkbar relativ zueinander gelagert sind, und eine Blockiereinrichtung aufweist, die in eine Freigabestellung und in eine Blockierstellung bringbar ist.

Derartige Gelenke sind beispielsweise aus der US 7,517,330 B2, der US 2006/0211966 A1 und der US 7,662,118 B2 bekannt. Sie werden beispielsweise als Gelenk für Orthesen, beispielsweise für Knieorthesen verwendet, können jedoch auch in anderen Orthesen oder orthopädietechnischen Einrichtungen Verwendung finden. Der erste Gelenkarm und der zweite Gelenkarm sind um eine Schwenkachse relativ zueinander schwenkbar gelagert. Dabei sind die über einen Schwenkbereich zu schwenken, der in aller Regel durch zwei Anschläge begrenzt wird, von denen jeweils einer die Bewegungsmöglichkeit in eine der beiden einander gegenüberliegenden Schwenkrichtungen begrenzt. Während sich die Blockiereinrichtung in der Freigabestellung befindet, können die beiden Gelenkarme in aller Regel frei zueinander in beide Schwenkrichtungen innerhalb des Schwenkbereichs verschwenkt werden. Befindet sich die Blockiereinrichtung jedoch in der Blockierstellung, wird dadurch eine Verschwenkung der beiden Gelenkarme relativ zueinander in eine erste Schwenkrichtung verhindert. Bei den Gelenken aus dem Stand der Technik wird dies beispielsweise dadurch erreicht, dass zwei Bauteile, die jeweils mit Ratschen oder schräg gestellten Zähnen versehen sind, ineinander eingreifen, wie dies aus der US 2007/0270976 A1 bekannt ist. Diese ratschenartigen Zähne verhindern eine weitere Verschwenkung in die erste Verschwenkrichtung, erlauben jedoch ein Verschwenken in die entgegengesetzte zweite Schwenkrichtung, da auf dieser Seite die Zähne abgeschrägt sind und aufeinander abgleiten können. Eine Verschwenkung der beiden Gelenkarme relativ zueinander in die zweite Schwenkrichtung, was auch mit der Blockiereinrichtung in der Blockierstellung möglich ist, endet jedoch dann, wenn einer der beiden Gelenkarme am zweiten Anschlag anschlägt, sodass eine weitere Verschwenkung auch in die zweite Schwenkrichtung nicht mehr möglich ist. In diesem Zustand ist das Gelenk vollständig blockiert und die beiden Gelenkarme können keine Schwenkbewegung mehr relativ zueinander ausführen.

Aus der US 8,715,367 B1 ist ein gattungsgemäßes Gelenk bekannt, bei dem die Blockiereinrichtung in der Blockierstellung ein Verschwenken der beiden Gelenkarme relativ zueinander in die erste Schwenkrichtung blockiert, unabhängig von dem Winkel, in dem sich die beiden Schwenkarme relativ zueinander befinden.

Ein gattungsgemäßes Gelenk kann beispielsweise in einer Knieorthese verwendet werden. Das Knie ist dann in aller Regel blockiert, da sich die Blockiereinrichtung standardmäßig in der Blockierstellung befindet und das Knie vollständig gestreckt ist, sodass einer der beiden Gelenkarme am zweiten Anschlag anliegt. Eine Bewegung der Gelenkarme in die erste Schwenkrichtung wird folglich durch die Blockiereinrichtung und eine Bewegung der Gelenkarme in die zweite Schwenkrichtung durch den jeweiligen zweiten Anschlag verhindert. Möchte sich der Träger einer derartigen Knieorthese jedoch beispielsweise hinsetzen, ist es von Vorteil, das Knie abwinkeln zu können. Um dies zu erreichen wird die Blockiereinrichtung aus der Blockierstellung in die Freigabestellung gebracht, sodass die beiden Gelenkarme relativ zueinander verschwenkt werden können. Anschließend wird die Blockiereinrichtung wieder in die Blockierstellung gebracht oder fällt von selbst in diese Stellung zurück. Steht der Träger einer Orthese mit einem derartigen Gelenk anschließend auf und streckt das Bein vollständig, sodass einer der beiden Gelenkarme am zweiten Anschlag anliegt, ist das Gelenk wieder vollständig blockiert und kann gefahrlos belastet werden. Falls der Träger der Orthese jedoch aufsteht und dabei das Gelenk beispielsweise nicht vollständig streckt ist es wichtig, beispielsweise im Falle der Belastung des Gelenkes, also für den Fall, dass auf einen der beiden Gelenkarme ein Drehmoment um die Schwenkachse wirkt, dafür zu sorgen, dass die Bewegung in die erste Schwenkrichtung, die einer Flexion des Gelenks entspricht, sicher verhindert wird. Im Stand der Technik wird dies, wie bereits dargelegt, über ineinander eingreifende Verzahnungen erreicht. Dies hat jedoch einerseits den Nachteil, dass nur bei bestimmten Winkelpositionen, nämlich dann, wenn die Zähne genau ineinandergreifen, eine tatsächliche Verrastung und Blockierung der Schwenkbewegung in die erste Schwenkrichtung erreicht wird.

Andererseits entsteht durch die aufeinander abgleitenden Zähne ein Ratschen oder Klackergeräusch, was als unangenehm und störend empfunden wird.

Der Erfindung liegt daher die Aufgabe zu Grunde, ein Gelenk gemäß dem Oberbegriff des Anspruchs 1 so zu verbessern, dass die genannten Nachteile vermieden oder zumindest abgeschwächt werden.

Die Erfindung löst die gestellte Aufgabe durch ein Gelenk nach dem Oberbegriff des Anspruchs 1, dass sich dadurch auszeichnet, dass die Blockiereinrichtung in der Blockierstellung die Verschwenkung des ersten Gelenkarmes relativ zu dem zweiten Gelenkarm in der ersten Schwenkrichtung unabhängig von einem Schwenkwinkel zwischen dem ersten Gelenkarm und dem zweiten Gelenkarm blockiert, sofern dieser Schwenkwinkel in einem vorbestimmten Bereich liegt, und die Verschwenkung in der ersten Schwenkrichtung erlaubt, sofern der Schwenkwinkel außerhalb des vorbestimmten Bereiches liegt. Anders als bei den Gelenken aus dem Stand der Technik ist damit eine Verschwenkung der beiden Gelenkarme relativ zueinander in der ersten Schwenkrichtung nicht nur an wenigen diskreten Stellen unterbunden, nämlich dann, wenn die Zähnungen der ineinander eingreifenden Bauteile genau ineinander passen, sondern unabhängig von dem Schwenkwinkel, der sich zwischen den beiden Gelenkarmen befindet, sofern dieser Schwenkwinkel in einem vorbestimmten Bereich liegt. Liegt der Schwenkwinkel außerhalb des vorbestimmten Bereiches wird die Bewegung der beiden Gelenkarme in der ersten Schwenkrichtung relativ zueinander nicht beeinträchtigt.

Dadurch wird der Tragekomfort einer orthopädietechnischen Einrichtung, die ein erfindungsgemäßes Gelenk beinhaltet, erhöht. Anders als im Stand der Technik, wo es noch zu einer Verschwenkung in der ersten Schwenkrichtung kommen kann, bis die Zähnungen der beiden ineinander eingreifenden Bauteile exakt aneinander anliegen, kann es bei einem erfindungsgemäßen Gelenk nicht zu dieser ungewollten kurzfristigen Verschwenkung kommen. Unabhängig von dem Schwenkwinkel zwischen den beiden Gelenkarmen wird erfindungsgemäß die weitere Verschwenkung in der ersten Schwenkrichtung immer verhindert, sofern der Schwenkwinkel innerhalb des vorbestimmten Bereiches liegt. Dadurch werden auch die aus dem Stand der Technik bekannten gegebenenfalls störenden Ratschen oder Klackergeräusche vermieden, sodass zum einen keine störenden Geräusche erzeugt werden und zum anderen eine orthopädietechnische Einrichtung, die beispielsweise unter regulärer Kleidung getragen wird, nicht oder nicht so schnell als orthopädietechnische Einrichtung erkannt werden kann.

Vorteilhafterweise ist eine Kontaktfläche an dem ersten Gelenkarm angeordnet, die mit einem Blockierelement in Kontakt kommen kann, das an dem zweiten Gelenkarm beweglich angeordnet ist. Dieser Kontakt kann dann stattfinden, wenn sich die Blockiereinrichtung in der Blockierstellung befindet. In einer bevorzugten Ausgestaltung wird durch den Kontakt des Blockierelementes mit der Kontaktfläche eine Verschwenkung des ersten Gelenkarmes relativ zu dem zweiten Gelenkarm in die erste Schwenkrichtung verhindert.

Dabei ist es durchaus möglich, dass das Blockierelement nur in dem vorbestimmten Bereich des Schwenkwinkels zwischen den beiden Gelenkarmen mit der Kontaktfläche in Kontakt kommen kann und bei Schwenkwinkeln, die nicht in dem vorbestimmten Bereich liegen, ein solcher Kontakt nicht erfolgt.

Die Kontaktfläche ist starr und unbeweglich am ersten Gelenkarm angeordnet. Dies bedeutet, dass sie jeder Bewegung des ersten Gelenkarmes folgt. Vorteilhafterweise verfügt der erste Gelenkarm über ein verbreitertes Endstück, das sich in einer bevorzugten Ausgestaltung um die Schwenkachse herum erstreckt. Die Kontaktfläche ist vorteilhafterweise an einer radial äußeren Umfangsfläche dieses Endstückes angeordnet.

Ist das Blockierelement als in einer Führung verschieblich gelagerter Nocken oder ähnliches verschieblich gelagertes Element ausgestaltet, kann das Blockierelement selbst über einen Anlagebereich verfügen, der zur Anlage an der Kontaktfläche vorgesehen ist. Dieser kann bevorzugt mit einer rutschhemmenden Beschichtung ausgebildet sein. Die Führung selbst kann tangential zu dem kreisförmig oder nahezu kreisförmig ausgebildeten verbreiterten Endstück des ersten Gelenkarmes ausgebildet sein, sodass sich zwischen der radial äußeren Kontaktfläche dieses Endstückes und der dieser Fläche abgewandten Innenseite der Führung zu einem Spalt kommt, in dem sich der Nocken befindet. Dieser Spalt weist dabei eine variable Breite auf und ist insbesondere so ausgebildet, dass er in eine Richtung abnimmt, während er in die andere Richtung zunimmt oder gleichbleibt. Dabei hat es sich als Vorteilhaft herausgestellt, wenn die Breite dieses Spaltes so weit abnimmt, dass sie kleiner ist als die Breite des verschieblich gelagerten Nockens.

Befindet sich nun die Blockiereinrichtung in der Blockierstellung, kommt der Nocken mit der Kontaktfläche in Kontakt. Durch eine weitere Verschwenkung des ersten Gelenkarmes relativ zum zweiten Gelenkarm in der ersten Schwenkrichtung würde der Nocken durch den Kontakt mit der Kontaktfläche der Bewegung der Kontaktfläche relativ zum zweiten Gelenkarm folgen und müssten in den Bereich des Spaltes bewegt werden, dessen Breite zu klein für den Nocken ist. Es kommt daher zu einer Klemmwirkung, die eine weitere Bewegung des Gelenkarms in der ersten Schwenkrichtung verhindert. Da die Breite des Spaltes in dieser Richtung abnimmt, handelt es sich zudem um einen selbstverstärkenden Effekt. Je stärker ein auf die Gelenkarme wirkendes Drehmoment ist, das eine Verschwenkung der beiden Gelenkarme relativ zueinander in der ersten Schwenkrichtung zur Folge hätte, desto stärker wird die Blockierwirkung der auf diese Weise ausgebildeten Blockiereinrichtung.

Eine Verschwenkung der beiden Gelenkarme in die entgegengesetzte zweite Schwenkrichtung ist jedoch problemlos möglich, da der Nocken durch den Kontakt mit der Kontaktfläche in die andere Richtung bewegt wird, in der sich der Spalt jedoch verbreitert oder in der die Breite des Spaltes konstant ist. Die Breite ist ausreichend, um den Nocken zu bewegen, sodass eine Verschwenkung in dieser Richtung problemlos möglich ist.

In einer alternativen Ausgestaltung ist das Blockierelement ein exzentrisch gelagerter Zapfen, Bolzen oder Zylinder. Das Blockierelement verfügt in dieser Ausgestaltung über eine Längsachse, parallel zu der es exzentrisch gelagert ist. Die Mantelfläche dieses Blockierelementes kommt mit der Kontaktfläche der Blockiereinrichtung in Kontakt, sofern sich die Blockiereinrichtung in der Blockierstellung befindet. Durch die exzentrische Lagerung des Blockierelementes ist folglich der Abstand der äußeren Mantelfläche des Blockierelementes von der Lagerachse über den Umfang nicht konstant, sondern weist ein Minimum und ein Maximum auf. Ändert sich nun der Abstand der Kontaktfläche, die am ersten Gelenkarm angeordnet ist, relativ zu dieser Lagerachse des Blockierelementes, rotiert das Blockierelement beispielsweise unter dem Einfluss der Schwerkraft um seine Lagerachse so lange, bis die Mantelfläche des Blockierelementes an der Kontaktfläche anliegt. In dieser Ausgestaltung ist es daher von Vorteil, wenn die Kontaktfläche am ersten Gelenkarm so ausgebildet ist, dass sie in unterschiedlichen Schwenkwinkeln, also zwischen dem ersten Gelenkarm und dem zweiten Gelenkarm eingeschlossenen Winkel, einen unterschiedlichen Abstand zur Lagerachse des Blockierelementes aufweist.

Vorzugsweise ist das Blockierelement in Richtung auf die Kontaktfläche vorgespannt. Vorzugsweise ist das Blockierelement durch ein Federelement oder die auf das Blockierelement wirkende Gewichtskraft vorgespannt. Selbstverständlich sind auch andere Möglichkeiten der Vorspannung möglich, die für unterschiedliche Ausgestaltungen vorteilhaft sind. Der Fachmann hat keine Schwierigkeiten, eine geeignete Wahl eines Vorspannungsmittels für eine bestimmte Form des Blockierelementes zu treffen.

Es hat sich als Vorteilhaft herausgestellt, wenn die Kontaktfläche bezüglich der Schwenkachse exzentrisch ausgebildet ist. Die bedeutet, dass der Abstand der Kontaktfläche relativ zur Schwenkachse über den Umfang nicht konstant ist.

Vorzugsweise verfügt das Gelenk über ein Betätigungselement, durch dessen Betätigung die Blockiereinrichtung aus der Blockierstellung in die Freigabestellung bringbar ist. Vorzugsweise erstreckt sich ein Schwenkbereich, in dem der erste Gelenkarm relativ zu dem zweiten Gelenkarm schwenkbar ist, von einem ersten Anschlag bis zu einem zweiten Anschlag, wobei der vorbestimmte Bereich kleiner ist als dieser Schwenkbereich. Dies bedeutet insbesondere, dass es einen Teil des Schwenkbereiches gibt, in dem auch dann eine Schwenkbewegung der beiden Gelenkarme relativ zueinander in der ersten Schwenkrichtung nicht blockiert ist, wenn sich die Blockiereinrichtung in der Blockierstellung befindet. Dies ist lediglich innerhalb des vorbestimmten Bereiches der Fall, der in diesem Ausführungsbeispiel kleiner ist als der Schwenkbereich. Insbesondere Vorzugsweise wird der vorbestimmte Bereich in einer zweiten Schwenkrichtung, die der ersten Schwenkrichtung entgegengesetzt ist, durch den zweiten Anschlag begrenzt. In diesem Fall ist es möglich, dass Gelenk vollständig zu blockieren, wenn sich die Blockiereinrichtung in der Blockierstellung befindet und der jeweilige Gelenkarm an dem zweiten Anschlag anliegt.

In einer bevorzugten Ausgestaltung verfügt das Gelenk zusätzlich über eine zweite Blockiereinrichtung, die in eine Freigabestellung und in eine Blockierstellung bringbar ist, in der sie die Verschwenkung des ersten Gelenkarmes relativ zu dem zweiten Gelenkarm in der zweiten Schwenkrichtung, die der ersten Schwenkrichtung entgegengesetzt ist, unabhängig von einem Schwenkwinkel zwischen dem ersten Gelenkarm und dem zweiten Gelenkarm blockiert, sofern dieser Schwenkwinkel in einem zweiten vorbestimmten Bereich liegt, und die Verschwenkung in der zweiten Schwenkrichtung erlaubt, sofern der Schwenkwinkel außerhalb des zweiten vorbestimmten Bereiches liegt.

Auf diese Weise wird ein Gelenk geschaffen, bei dem für jede Schwenkrichtung zwei Bereiche vorhanden sind. In einem der beiden Bereiche ist die Verschwenkung in die jeweilige Schwenkrichtung durch die Blockiereinrichtung erlaubt, im anderen wird sie unterbunden. Da jede Blockiereinrichtung vorteilhafterweise die Bewegung in die jeweils andere Schwenkrichtung nicht beeinträchtigt, lässt sich auf diese Weise die Verschwenkbarkeit der beiden Gelenkarme nahezu völlig frei einstellen.

Über die gewählte Kontur der Kontaktfläche, die mit dem Blockierelement der jeweiligen Blockiereinrichtung in Kontakt kommt, um eine weitere Verschwenkung in einer der beiden Schwenkrichtungen zu vermeiden, lässt sich der vorbestimmte Bereich und der zweite vorbestimmte Bereich individuell einstellen. Prinzipiell ist es auch möglich, für eine Blockiereinrichtung mehrere Bereiche vorzusehen, in denen beispielsweise eine Verschwenkung erlaubt ist.

Mit Hilfe der beigefügten Zeichnungen wird nachfolgend ein Ausführungsbeispiel der vorliegenden Erfindung näher erläutert. Es zeigt
- Figur 1 -: die schematische Draufsicht auf ein Gelenk gemäß einem ersten Ausführungsbeispiel der vorliegenden Erfindung,
- Figuren 2 bis 5 -: eine Schnittdarstellung durch das in Figur 1 gezeigte Gelenk in unterschiedlichen Stellungen,
- Figuren 6a bis 6c -: jeweils zwei Ansichten eines Gelenkes gemäß einem weiteren Ausführungsbeispiel der vorliegenden Erfindung in unterschiedlichen Stellungen,
- Figur 7 -: die schematische Darstellung eines Gelenkes gemäß einem weiteren Ausführungsbeispiel,
- Figur 8 -: die Darstellung eines weiteren Gelenkes gemäß einem Ausführungsbeispiel der vorliegenden Erfindung und
- Figur 9a und 9b -: zwei Ansichten eines Gelenkes gemäß einem weiteren Ausführungsbeispiel der vorliegenden Erfindung.

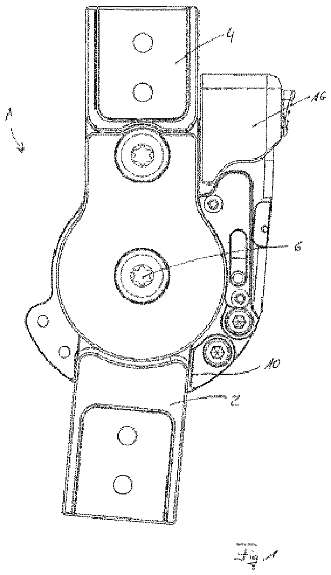

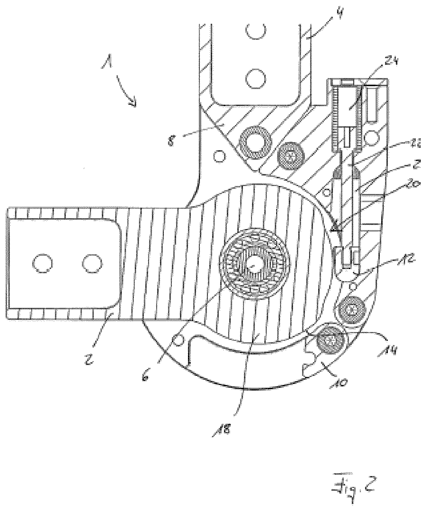

Figur 1 zeigt ein Gelenk 1 gemäß einem ersten Ausführungsbeispiel der vorliegenden Erfindung. Es verfügt über einen ersten Gelenkarm 2 und einen zweiten Gelenkarm 4, die um eine Schwenkachse 6 schwenkbar relativ zueinander gelagert sind. Der Schwenkbereich, über den eine derartige Verschwenkung möglich ist, wird durch einen in Figur 1 nicht dargestellten ersten Anschlag 8 und einen zweiten Anschlag 10 begrenzt. Im Inneren des in Figur 1 dargestellten Gelenkes 1 befindet sich ein Blockierelement 12 und eine Kontaktfläche 14, die in den Figuren 2 bis 5 dargestellt sind. Über ein Betätigungselement 16 kann das Blockierelement 12 in seiner Lage geändert werden, wodurch die Blockiereinrichtung aus der Blockierstellung in die Freigabestellung gebracht wird. Dazu muss im gezeigten Ausführungsbeispiel das Betätigungselement 16 in Figur 1 nach oben verschoben werden.

Die Figuren 2 bis 5 zeigen eine Schnittdarstellung durch das in Figur 1 gezeigte Gelenk 1. In Figur ist der erste Gelenkarm 2 relativ zum zweiten Gelenkarm 4 im Vergleich zu Figur verschwenkt worden. Der erste Gelenkarm 2 verfügt über ein Endstück 18, das sich um die Schwenkachse 6 herum erstreckt. Im radial äußeren Bereich dieses Endstückes 18 befindet sich die Kontaktfläche 14, die in der in Figur 2 gezeigten Situation außer Eingriff mit dem Blockierelement 12 ist.

Der radiale Abstand der Kontaktfläche 14 von der Schwenkachse 6 ist über den Umfang, wie in Figur 2 deutlich zu erkennen ist, nicht konstant. Das Endstück 18 verfügt über einen abgeflachten Bereich 20, in dem der Abstand der Kontaktfläche 14 von der Schwenkachse 6 kleiner ist als im übrigen Bereich. Daher ist die Kontaktfläche 14 in der in Figur 2 gezeigten Winkelstellung der beiden Gelenkarme 2, 4 relativ zueinander nicht mit dem Blockierelement 12 in Eingriff.

Das Gelenk 1 befindet sich folglich in einer Position, indem eine Verschwenkung des ersten Gelenkarmes 2 relativ zum zweiten Gelenkarm 4 sowohl in die erste Schwenkrichtung als auch in die zweite Schwenkrichtung möglich ist, obwohl sich die Blockiereinrichtung in der Blockierstellung befindet.

Das Blockierelement 12 befindet sich an einem Stift 22, der durch eine Feder 24 belastet ist, die ihn in Figur 2 nach unten drückt. Das Blockierelement 2 wird folglich ebenfalls nach unten gedrückt, sodass die Blockiereinrichtung in die Blockierstellung vorbelastet ist. Über das in Figur 2 nicht dargestellte Betätigungselement 16 kann der Stift 22 nach oben verschoben werden, wodurch auch das Blockierelement 12 innerhalb einer Führung 26 nach oben verschoben wird. Auf diese Weise kann die Blockiereinrichtung aus der Blockierstellung in die Freigabestellung gebracht werden.

Man erkennt in Figur 2 neben dem zweiten Anschlag 10 auch den ersten Anschlag 8, die die mögliche Schwenkbewegung begrenzt. Über die Formgebung des ersten Anschlags 8 und des zweiten Anschlags 10 kann der maximale Schwenkbereich eingeschränkt werden.

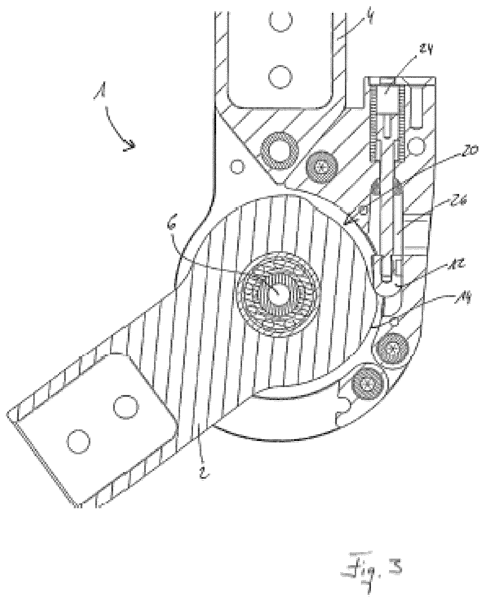

In Figur 3 ist der erste Gelenkarm 2 relativ zum zweiten Gelenkarm 4 in Richtung Extension des Gelenks 1 verschwenkt worden. Die Kontaktfläche 14 guckt nun mit einem Bereich, in dem der Abstand zwischen der Kontaktfläche 14 und der Schwenkachse 6 deutlich größer ist als im abgeflachten Bereich 20 mit dem Blockierelement 12 in Kontakt. Dadurch wird das Blockierelement 12 im Vergleich zur Figur 2 nach oben verschoben, wodurch die Feder 24 komprimiert wird. Diese Verschiebung ist gegen die Kraft der Feder 24 jedoch möglich, sodass eine Verschwenkung des ersten Gelenkarmes 2 relativ zum zweiten Gelenkarm 4 in Richtung Extension möglich ist. Bei diesem in Figur 3 gezeigten Schwenkwinkel zwischen dem ersten Gelenkarm 2 und dem zweiten Gelenkarm 4 wäre auch eine weitere Verschwenkung des ersten Gelenkarmes 2 relativ zum zweiten Gelenkarm 4 in Richtung Flexion weiterhin möglich, da das Blockierelement 12 nach unten in der Führung 26 bewegt werden kann.

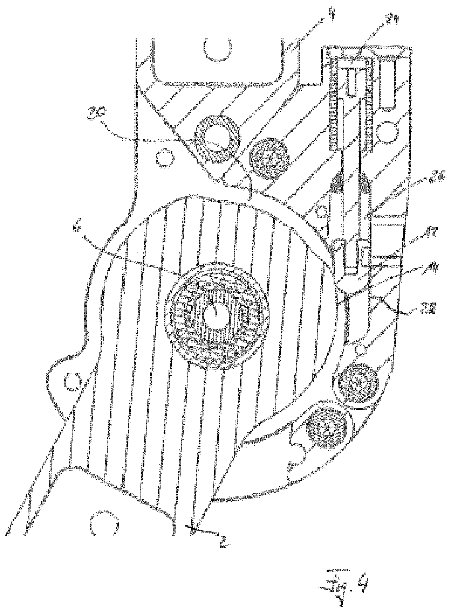

Figur 4 zeigt die Situation, in der der erste Gelenkarm 2 relativ zum zweiten Gelenkarm 4 noch weiter in Richtung Extension verschwenkt wurde. Die Kontaktfläche 14 ist nun mit dem Blockierelement 12 in Kontakt, das in der Führung 26 weiter gegen die Kraft der nun stark komprimierten Feder 24 verschoben wurde. Der abgeflachte Bereich 20 bietet nun keinen Bewegungsspielraum für das Blockierelement 12 mehr. Der Abstand zwischen der Kontaktfläche 14 und der Schwenkachse 6 ist in dem Bereich, in dem die Kontaktfläche 14 mit dem Blockierelement 12 in Kontakt kommt, so groß, dass ein zwischen der Kontaktfläche 14 und einer äußeren Wand 28 der Führung 26 entstehende Schlitz nicht breit genug ist, um das Blockierelement 12 aufzunehmen.

Eine weitere Verschwenkung des ersten Gelenkarmes 2 relativ zum zweiten Gelenkarm 4 in Richtung Extension ist daher möglich, da das Blockierelement 12 weiter in der Führung 26 nach oben verschoben werden kann. Eine entgegengesetzte Verschwenkung des ersten Gelenkarmes 2 relativ zum zweiten Gelenkarm 4 in Richtung Flexion wird jedoch durch die Blockiereinrichtung blockiert, da durch den Kontakt der Kontaktfläche 14 mit dem Blockierelement 12 bei dieser Verschwenkung das Blockierelement 12 in den zu engen Schlitz zwischen Kontaktfläche 14 und äußerer Wand 28 verschoben werden müsste. Dies ist jedoch aufgrund der zu großen Breite des Blockierelementes 12 in dieser Richtung nicht möglich, weswegen die Bewegung blockiert ist.

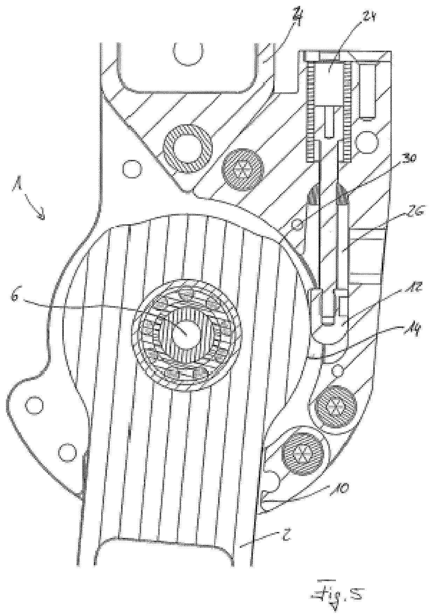

Figur 5 zeigt das Gelenk 1 in nahezu vollständiger Extension. Der erste Gelenkarm 2 liegt nahezu am zweiten Anschlag 10 an, der eine weitere Verschwenkung des ersten Gelenkarmes 2 relativ zum zweiten Gelenkarm 4 in Richtung Extension verhindert. Die Kontaktfläche 14 ist weiterhin mit dem Blockierelement 12 in Kontakt und verhindert auf die bereits zu Figur 4 beschriebene Weise eine Verschwenkung des ersten Gelenkarmes 2 relativ zum zweiten Gelenkarm 4 in Flexionsrichtung. Man erkennt im Vergleich zu Figur 5, dass das Blockierelement 12 in der Führung 26 nach unten verschoben ist. Die Feder 24 ist gegenüber der in Figur 4 gezeigten Situation deutlich entspannt. Dies wird erreicht, indem die Kontaktfläche 14 exzentrisch ausgebildet ist. Dies bedeutet, dass auch in dem Bereich, in dem die Kontaktfläche 14 mit dem Blockierelement 12 in Kontakt kommen kann, der Abstand zwischen der Kontaktfläche 14 und der Schwenkachse 6 nicht konstant ist. Vielmehr nimmt der im gezeigten Ausführungsbeispiel von einer Maximalstelle 30 ausgehend, an der der Abstand zwischen der Kontaktfläche 14 und der Schwenkachse 6 maximal ist, kontinuierlich ab. In der gezeigten Situation in Figur 5 ist das Gelenk 1 nahezu vollständig blockiert, da eine Verschwenkung des ersten Gelenkarms 2 relativ zum zweiten Gelenkarm 4 in Extensionsrichtung durch den zweiten Anschlag 10 und in Flexionsrichtung durch das Blockierelement 12 verhindert wird.

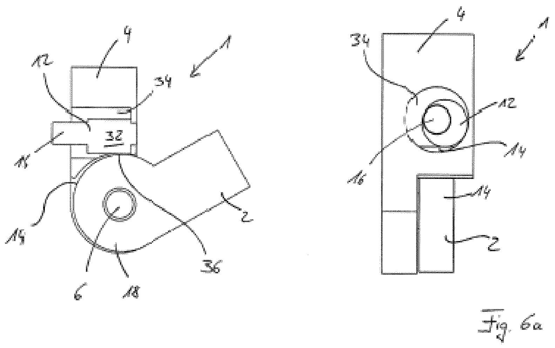

Die Figuren 6a bis 6c zeigen eine weitere Ausführungsform des Gelenkes 1. Dabei zeigt die jeweils linke Darstellung der Figuren eine Schnittdarstellung und die jeweils rechte Darstellung eine Seitenansicht des Gelenkes 1. Es verfügt über den ersten Gelenkarm 2, den zweiten Gelenkarm 4 und die Schwenkachse 6. Das Endstück 18 des ersten Gelenkarms 2 verfügt an der radial äußeren Seite über die Kontaktfläche 14, und erstreckt sich um die Schwenkachse 6 herum. Anders als im in den Figuren 1 bis 5 gezeigten Ausführungsbeispiel ist das Blockierelement 12 hier als exzentrisch gelagerter Zylinder dargestellt. Er verfügt über eine Mantelfläche 32 und ist exzentrisch gelagert, wie dies beispielsweise in den rechten Darstellungen der Figuren 6a bis 6c gezeigt ist. Zentral in der Verlängerung der Lagerachse dieser exzentrischen Lagerung befindet sich das Betätigungselement 16 das im gezeigten Ausführungsbeispiel ausgebildet ist, das Blockierelement 12 um die Längsachse des Betätigungselementes, die im gezeigten Ausführungsbeispiel gleichzeitig die Lagerachse auch des Blockierelementes 12 ist, zu drehen.

Das Blockierelement befindet sich in einer Ausnehmung 34, die eine derartige Drehung erlaubt und an ihrer Unterseite eine Öffnung 36 aufweist, durch die die Kontaktfläche 14 in die Ausnehmung 34 hineinragen kann.

Auch in dem Ausführungsbeispiel des Gelenkes 1, das in den Figuren 6a bis 6c dargestellt ist, ist die Kontaktfläche 14 exzentrisch ausgebildet. Dies bedeutet wieder, dass der Abstand zwischen der Kontaktfläche 14 und der Schwenkachse 6 über den Umfang des Endstücks 18 nicht konstant ist.

In der in Figur 6a gezeigten Situation ist das Gelenk 1 stark gebeugt. Es handelt sich um die nahezu vollständige Flexion des Gelenkes 1. Die Kontaktfläche 14 ist so ausgebildet, dass sie in dieser Position möglichst weit in die Ausnehmung 34 hineinragt, wie dies insbesondere in der rechten Darstellung der Figur 6a zu erkennen ist. Die Mantelfläche 32 des Blockierelementes 12 liegt an der Kontaktfläche 14 an.

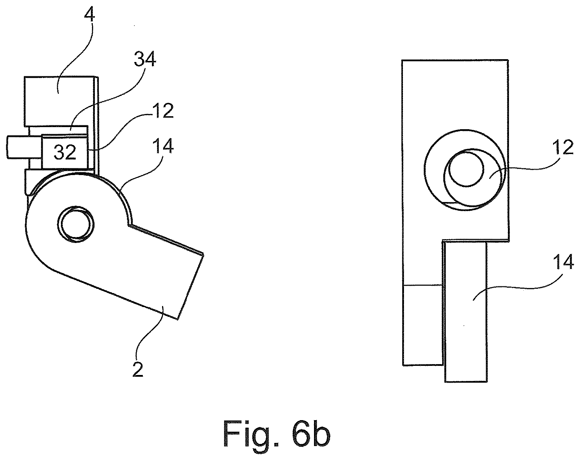

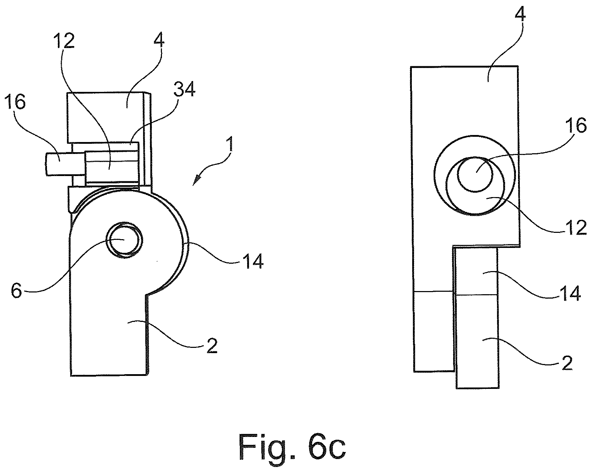

In Figur 6b ist in den beiden Darstellungen eine Situation gezeigt, in der der erste Gelenkarm 2 relativ zum zweiten Gelenkarm 4 in Richtung Extension verschwenkt wurde. Aufgrund der Exzentrizität der Kontaktfläche 14 ragt diese nun nicht mehr so weit in die Ausnehmung 34 hinein. Man erkennt, dass dadurch das Blockierelement 12 um die Lagerachse herum gedreht wurde, da die Mantelfläche 32 weiterhin an der Kontaktfläche 14 anliegt. Figur 6c zeigt das Gelenk 1 in vollständiger Extension. Der erste Gelenkarm 2 ist relativ zum zweiten Gelenkarm 4 noch weiter verschoben worden, wodurch die Kontaktfläche 14 nun gar nicht mehr oder nur noch minimal in die Ausnehmung 34 hineinragt. Man erkennt insbesondere in der rechten Darstellung der Figur 4c, dass das Blockierelement 12 noch weiter um die Lagerachse, die gleichzeitig die Längsachse des Betätigungselementes 16 ist, verdreht wurde. Diese Drehung des Blockierelementes 12 erfolgt im gezeigten Ausführungsbeispiel allein aufgrund der Schwerkraft. Selbstverständlich kann hier auch ein Kraftaufbringelement vorhanden sein, das das Blockierelement 12 in die in Figur 6c gezeigte Position vorspannt.

Eine Verschwenkung des ersten Gelenkarms 2 relativ zum zweiten Gelenkarm 4 in der in Figur 6c gezeigten Situation in Flexionsrichtung, also gegen den Uhrzeigersinn um die Schwenkachse 6, wird durch das Blockierelement 12 verhindert. Eine derartige Verschwenkung hätte zur Folge, dass die Kontaktfläche 14 das Blockierelement 12 nach oben drückt. Dies ist jedoch aufgrund der Lagerung des Blockierelementes 12 nicht möglich, da es mit einer Verschwenkung des Blockier-elementes 12 um die Längsachse des Betätigungselementes 16 einhergehen müsste, die durch die Verschwenkung der beiden Gelenkarme 2, 4, relativ zueinander nicht zu erreichen ist.

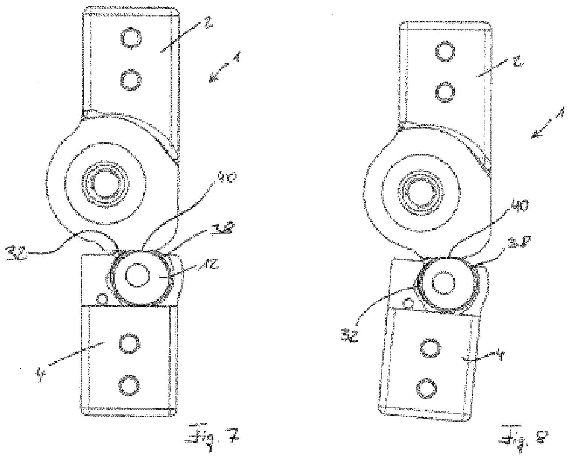

Figur 7 zeigt ein weiteres Gelenk 1 mit dem ersten Gelenkarm 2 und dem zweiten Gelenkarm 4. Am zweiten Gelenkarm 4 ist das Blockierelement 12 zu erkennen, dessen Mantelfläche 32 mit der in Figur 7 nicht dargestellten Kontaktfläche 14 des Endstücks 18 des ersten Gelenkarmes 2 zusammenwirkt. In Figur 7 ist zu erkennen, dass das Blockierelement 12 eine Anschlagsmantelfläche 38 aufweist, die ebenfalls exzentrisch zur Rotationsachse des Blockierelementes 12 angeordnet ist. Über die Positionierung dieser Anschlagsmantelfläche 38 relativ zur Mantelfläche 32, die mit der Kontaktfläche 14 in Kontakt kommt, lässt sich der Anschlag des Gelenkes 1, also die maximale Extension einstellen. Man erkennt, dass ein Gelenkanschlag 40 am ersten Gelenkarm 2 in Figur 7 an der Anschlagsmantelfläche 38 anliegt und so ein weiteres Verschwenken der beiden Gelenkarme 2, 4 in Extension relativ zueinander verhindert.

Figur 8 zeigt das Gelenk aus Figur 7, bei dem die Anschlagsmantelfläche 38 relativ zur Mantelfläche 32 verdreht ausgebildet ist. Dadurch wird erreicht, dass der Gelenkanschlag 40 bei einem anderen Winkel zwischen den beiden Gelenkarmen 2, 4 an der Anschlagsmantelfläche 38 anschlägt und somit die Extension des Gelenkes 1 in Extensionsrichtung begrenzt. Im gezeigten Ausführungsbeispiel ist der Anschlagswinkel des in Figur 8 gezeigten Gelenkes 1 gegenüber dem in Figur 7 gezeigten Gelenkes 1 um 5° verschoben.

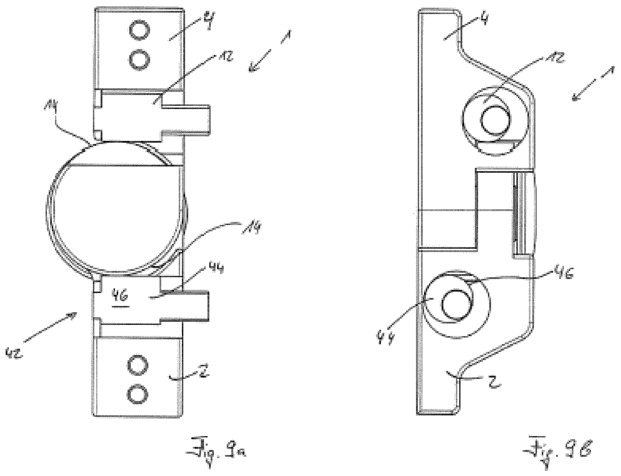

Die Figuren 9a und 9b zeigen jeweils eine Ansicht eines Gelenkes 1 das über das Blockierelement 12 in der bereits beschriebenen Ausführungsform verfügt. Zusätzlich verfügt das Gelenk 1 jedoch über eine zweite Blockiereinrichtung 42, die über ein zweites Blockierelement 44 verfügt. Es verfügt über eine zweite Mantelfläche 46, die mit der Kontaktfläche 40 in Kontakt ist und zusammenwirkt. Alternativ zu der gezeigten Ausführungsform können auch zwei separate Kontaktflächen 14 vorhanden sein. Während die erste Blockiereinrichtung mit dem Blockierelement 12 ein Verschwenken der beiden Gelenkarme 2, 4 in die erste Schwenkrichtung verhindert, sofern der Schwenkwinkel zwischen den beiden Gelenkarmen 2, 4 innerhalb eines vorbestimmten Bereiches liegt, verhindert die zweite Blockiereinrichtung 42 ein Verschwenken der beiden Gelenkarme 2, 4 in die zweite Schwenkrichtung, die der ersten Schwenkrichtung gegenüberliegt. Eine Verschwenkung in diese zweite Schwenkrichtung wird jedoch nur verhindert, sofern der Schwenkwinkel sich innerhalb des zweiten vorbestimmten Bereiches befindet.

In Figur 9b ist zu erkennen, dass die beiden Blockierelemente 12, 44 versetzt zueinander angeordnet sind. Auf diese Weise lässt sich besonders einfach die Positionierung zweier Kontaktflächen 14 erreichen, von denen eine am ersten Gelenkarm 2 und die zweite am zweiten Gelenkarm 4 angeordnet ist. Über die Auswahl der Form der Kontur dieser Kontaktflächen 14 lässt sich das Schwenkverhalten des Gelenkes 1 bestimmen.

### Bezugszeichenliste

- 1: Gelenk
- 2: erster Gelenkarm
- 4: zweiter Gelenkarm
- 6: Schwenkachse
- 8: erster Anschlag
- 10: zweiter Anschlag
- 12: Blockierelement
- 14: Kontaktfläche
- 16: Betätigungselement
- 18: Endstück
- 20: abgeflachter Bereich
- 22: Stift
- 24: Feder
- 26: Führung
- 28: äußere Wand
- 30: Maximalstelle
- 32: Mantelfläche
- 34: Ausnehmung
- 36: Öffnung
- 38: Anschlagsmantelfläche
- 40: Gelenkanschlag
- 42: zweite Blockiereinrichtung
- 44: zweites Blockierelement
- 46: zweite Mantelfläche

## Patentansprüche

1. Gelenk (1) für eine orthopädietechnische Einrichtung, das
einen ersten Gelenkarm (2) und einen zweiten Gelenkarm (4),
die um eine Schwenkachse (6) schwenkbar relativ zueinander gelagert sind, und
eine Blockiereinrichtung aufweist,
die in eine Freigabestellung und in eine Blockierstellung bringbar ist,
wobei die Blockiereinrichtung in der Blockierstellung die Verschwenkung des ersten Gelenkarmes (2) relativ zu dem zweiten Gelenkarm (4) in der ersten Schwenkrichtung unabhängig von einem Schwenkwinkel zwischen dem ersten Gelenkarm (2) und dem zweiten Gelenkarm (4) blockiert, sofern dieser Schwenkwinkel in einem vorbestimmten Bereich liegt und die Verschwenkung in der ersten Schwenkrichtung erlaubt, sofern der Schwenkwinkel außerhalb des vorbestimmten Bereiches liegt.

2. Gelenk (1) nach Anspruch 1, wobei eine Kontaktfläche (14) an dem ersten Gelenkarm (2) angeordnet ist, die mit einem Blockierelement (12), das an dem zweiten Gelenkarm (4) beweglich angeordnet ist, in Kontakt kommt, wenn sich die Blockiereinrichtung in der Blockierstellung befindet.

3. Gelenk (1) nach Anspruch 2, wobei den Kontakt des Blockierelementes (12) mit der Kontaktfläche (14) eine Verschwenkung des ersten Gelenkarms (2) relativ zu dem zweiten Gelenkarm (4) in die erste Schwenkrichtung verhindert wird.

4. Gelenk (1) nach Anspruch 2 oder 3, wobei das Blockierelement ein in einer Führung (26) verschieblich gelagerter Nocken oder ein exzentrisch gelagerter Zapfen, Bolzen oder Zylinder ist.

5. Gelenk (1) nach einem der Ansprüche 2 bis 4, wobei das Blockierelement (12) in Richtung auf die Kontaktfläche (14) vorgespannt ist.

6. Gelenk (1) nach Anspruch 5, wobei das Blockierelement (12) durch ein Federelement (24) oder die auf das Blockierelement (12) wirkende Gewichtskraft vorgespannt ist.

7. Gelenk (1) nach einem der Ansprüche 2 bis 6, wobei die Kontaktfläche (14) bezüglich der Schwenkachse (6) exzentrisch ausgebildet ist.

8. Gelenk (1) nach einem der vorstehenden Ansprüche, wobei das Gelenk (1) ein Betätigungselement (16) aufweist, durch dessen Betätigung die Blockiereinrichtung aus der Blockierstellung in die Freigabestellung bringbar ist.

9. Gelenk (1) nach einem der vorstehenden Ansprüche, wobei sich ein Schwenkbereich, in dem der erste Gelenkarm (2) relativ zu dem zweiten Gelenkarm (4) schwenkbar ist, von einem ersten Anschlag (8) bis zu einem zweiten Anschlag (10) erstreckt, wobei der vorbestimmte Bereich kleiner als der Schwenkbereich ist und wobei insbesondere der vorbestimmte Bereich in einer zweiten Schwenkrichtung, die der ersten Schwenkrichtung entgegengesetzt ist, durch den zweiten Anschlag (10) begrenzt wird.

10. Gelenk (1) nach einem der vorstehenden Ansprüche, wobei **das** Gelenk (1) zusätzlich eine zweite Blockiereinrichtung (42) aufweist, die in eine Freigabestellung und eine Blockierstellung bringbar ist, in der sie die Verschwenkung des ersten Gelenkarmes (2) relativ zu dem zweiten Gelenkarm (4) in der zweiten Schwenkrichtung, die der ersten Schwenkrichtung entgegengesetzt ist, unabhängig von dem Schwenkwinkel zwischen dem ersten Gelenkarm (2) und dem zweiten Gelenkarm (4) blockiert, sofern dieser Schwenkwinkel in einem zweiten vorbestimmten Bereich liegt und die Verschwenkung in der zweiten Schwenkrichtung erlaubt, sofern der Schwenkwinkel außerhalb des zweiten vorbestimmten Bereichs liegt.

## Claims

1. Joint (1) for an orthopedic device, the joint having
a first articulated arm (2) and a second articulated arm (4),
which are mounted about a swivel axis (6) such that they can be swiveled relative to one another, and
a blocking device,
which can be moved into a release position and a blocking position,
wherein the blocking device, when in the blocking position, blocks the swiveling of the first articulated arm (2) relative to the second articulated arm (4) in the first swivel direction, independently of a swivel angle between the first articulated arm (2) and the second articulated arm (4), when said swivel angle is in a predetermined range, and allows the swiveling in the first swivel direction, when the swivel angle is outside of the predetermined range.

2. Joint (1) according to claim 1, wherein a contact surface (14) is arranged on the first articulated arm (2) and comes into contact with a blocking element (12), which is movably arranged on the second articulated arm (4), when the blocking device is in the blocking position.

3. Joint (1) according to claim 2, wherein the contact between the blocking element (12) and the contact surface (14) prevents a swiveling of the first articulated arm (2) relative to the second articulated arm (4) in the first swivel direction.

4. Joint (1) according to claim 2 or 3, wherein the blocking element is a cam mounted in a guide (26) such that it can be moved or an eccentrically mounted pin, bolt or cylinder.

5. Joint (1) according to one of the claims 2 to 4, wherein the blocking element (12) is preloaded towards the contact surface (14).

6. Joint (1) according to claim 5, wherein the blocking element (12) is preloaded by a spring (24) or the weight force acting on the blocking element (12).

7. Joint (1) according to one of the claims 2 to 6, wherein the contact surface (14) is designed to be eccentric relative to the swivel axis (6).

8. Joint (1) according to one of the above claims, wherein the joint (1) comprises an activation element (16), by the activation of which the blocking device is movable from the blocking position into the release position.

9. Joint (1) according to one of the above claims, wherein a swivel range in which the first articulated arm (2) can be swiveled relative to the second articulated arm (4) extends from a first limit stop (8) to a second limit stop (10), wherein the predetermined range is smaller than the swivel range and wherein, in particular, the predetermined range in a second swivel direction is restricted by the second limit stop (10), this second swivel direction being the opposite direction to the first swivel direction.

10. Joint (1) according to one of the above claims, wherein the joint (1) also has a second blocking device (42) which can be moved into a release position and a blocking position, in which it blocks the swiveling of the first articulated arm (2) relative to the second articulated arm (4) in the second swivel direction, which is opposite to the first swivel direction, independently of a swivel angle between the first articulated arm (2) and the second articulated arm (4), when said swivel angle is in a predetermined range, and allows the swiveling in the second swivel direction, when the swivel angle is outside of the predetermined range.

## Revendications

1. Articulation (1) pour un dispositif orthopédique, laquelle comprend
un premier bras articulé (2) et un deuxième bras articulé (4) qui sont montés de manière à pouvoir pivoter l'un par rapport à l'autre autour d'un axe de pivotement (6), et
un dispositif de blocage qui peut être amené dans une position de déblocage et dans une position de blocage,
dans laquelle
dans la position de blocage, le dispositif de blocage empêche le pivotement du premier bras articulé (2) par rapport au deuxième bras articulé (4) dans la première direction de pivotement, indépendamment d'un angle de pivotement entre le premier bras articulé (2) et le deuxième bras articulé (4) dans la mesure où cet angle de pivotement est dans une plage prédéterminée, et autorise le pivotement dans la première direction de pivotement dans la mesure où l'angle de pivotement est hors de la plage prédéterminée.

2. Articulation (1) selon la revendication 1,
dans laquelle une surface de contact (14) est disposée sur le premier bras articulé (2) et vient en contact avec un élément de blocage (12) disposé de manière mobile sur le deuxième bras articulé (4) lorsque le dispositif de blocage se situe dans la position de blocage.

3. Articulation (1) selon la revendication 2,
dans laquelle le contact de l'élément de blocage (12) avec la surface de contact (14) permet d'empêcher le pivotement du premier bras articulé (2) par rapport au deuxième bras articulé (4) dans la première direction de pivotement.

4. Articulation (1) selon la revendication 2 ou 3,
dans laquelle l'élément de blocage est une came montée coulissante dans un guide (26) ou est un tourillon, un boulon ou un cylindre monté de manière excentrée.

5. Articulation (1) selon l'une des revendications 2 à 4,
dans laquelle l'élément de blocage (12) est précontraint en direction de la surface de contact (14).

6. Articulation (1) selon la revendication 5,
dans laquelle l'élément de blocage (12) est précontraint par un élément élastique (24) ou par le poids agissant sur l'élément de blocage (12).

7. Articulation (1) selon l'une des revendications 2 à 6,
dans laquelle la surface de contact (14) est excentrée par rapport à l'axe de pivotement (6).

8. Articulation (1) selon l'une des revendications précédentes,
dans laquelle l'articulation (1) comporte un élément d'actionnement (16) dont l'actionnement permet de faire passer le dispositif de blocage de la position de blocage à la position de déblocage.

9. Articulation (1) selon l'une des revendications précédentes,
dans laquelle une plage de pivotement, dans laquelle le premier bras articulé (2) peut pivoter par rapport au deuxième bras articulé (4), s'étend d'une première butée (8) à une deuxième butée (10), la plage prédéterminée étant plus petite que la plage de pivotement et la plage prédéterminée étant en particulier limitée par la deuxième butée (10) dans une deuxième direction de pivotement qui est opposée à la première direction de pivotement.

10. Articulation (1) selon l'une des revendications précédentes,
dans laquelle l'articulation (1) comporte en outre un deuxième dispositif de blocage (42) qui peut être amené dans une position de déblocage et dans une position de blocage dans laquelle il empêche le pivotement du premier bras articulé (2) par rapport au deuxième bras articulé (4) dans la deuxième direction de pivotement qui est opposée à la première direction de pivotement, indépendamment de l'angle de pivotement entre le premier bras articulé (2) et le deuxième bras articulé (4) dans la mesure où cet angle de pivotement est dans une deuxième plage prédéterminée, et autorise le pivotement dans la deuxième direction de pivotement dans la mesure où l'angle de pivotement est hors de la deuxième plage prédéterminée.
